# EUROPEAN PATENT APPLICATION

(11) **EP 4 335 434 A1**
(43) Date of publication of application: **13.03.2024**
(21) Application number: 23191571.1
(22) Date of filing: 16.08.2023
(51) Int. Cl.: A61K 9/16, A61K 9/20, A61K 31/496, A61P 11/06

(54) **PHARMACEUTICAL COMPOSITIONS COMPRISING IVACAFTOR**

(30) Priority: 17.08.2022 TR 202212995
(71) Applicant: SANOVEL ILAC SANAYI VE TICARET A.S., Istanbul 34460 (TR)
(72) Inventor: GULTEKIN INCE, Ozlem, Istanbul (TR); ARI, Busra, Istanbul (TR)
(74) Representative: Sevinç, Erkan

(57) **Abstract**

The present invention relates to a solid pharmaceutical composition comprising a solid dispersion of Ivacaftor wherein the Ivacaftor is dispersed in a matrix containing at least two polymers. The pharmaceutical composition is obtained by using an effective process.

## Description

### Field of the Invention

The present invention relates to a solid pharmaceutical composition comprising a solid dispersion of Ivacaftor wherein the Ivacaftor is dispersed in a matrix containing at least two polymers. The pharmaceutical composition is obtained by using an effective process.

### Background of the Invention

Ivacaftor is classified as a cystic fibrosis transmembrane conductance regulator (CFTR) potentiator and is indicated for the treatment of cystic fibrosis in patients who have a G55ID mutation in the CFTR gene.

Ivacaftor is a white powder that is practically insoluble in water (< 0.05 microgram/mL) and its having an empirical formula of C₂₄H₂₈N₂O₃ and a molecular weight of 392.499 g/moL. Ivacaftor chemically known as N-(2,4-di-tert-butyl-5-hydroxyphenyl)-1,4-dihydro-4-oxoquinoline-3-carboxamide and its chemical structure is shown in the Formula I.

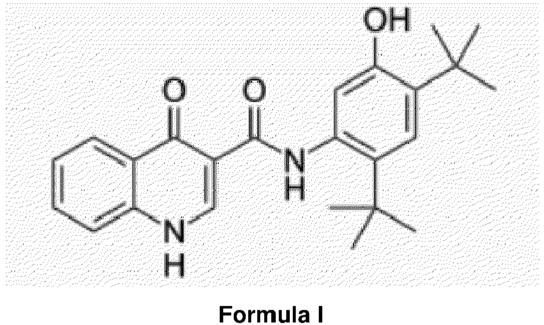

Ivacaftor was approved by FDA in 2012 and developed by VERTEX^{®} pharmaceuticals in amorphous solid dispersion tablets form under the trade name Kalydeco for the treatment of cystic fibrosis. Ivacaftor is currently marketed as tablets in strength of 75 mg and 150 mg and oral granules in strength of 25 mg, 50 mg and 75 mg per packet under the trade name Kalydeco and is indicated for the treatment of cystic fibrosis in patients age 6 years and older who have a G55ID mutation in the CFTR (cystic fibrosis transmembrane conductance regulator) gene.

Ivacaftor has low water solubility, very lipophilic and bioavailability of Ivacaftor is significantly enhanced when co-administered with food. Kalydeco is administered orally and it is recommended with a fatty meal to aid in its stomach absorption to improve its bioavailability and overall exposure.

Ivacaftor molecule has been disclosed in WO2006002421 patent. WO2007079139 disclosed amorphous solid dispersion with Ivacaftor and polymer. In this patent, the amount of Ivacaftor in the solid dispersion is 50% and the amount of polymer is 50%.

WO2010019239 patent discloses that this amorphous solid dispersion was improved reducing the quantity of polymer until about 20% and increasing the quantity of ivacaftor until 80% in the solid dispersion.

WO2007134279 patent discloses a pharmaceutical composition comprising Ivacaftor, PEG 400 and PVP K30.

Accordingly, there is a need for stable, bioavailable and high solubility pharmaceutical compositions of Ivacaftor useful for treating patients suffering from cystic fibrosis.

Hence, it would be desirable to have a pharmaceutical composition comprising Ivacaftor that is stable, having a good content uniformity and exhibits excellent dissolution profile and high bioavailability.

### Detailed Description of the Invention

The present invention relates to a pharmaceutical composition comprising a solid dispersion of Ivacaftor or pharmaceutically acceptable salt thereof and method of manufacturing.

The main object of the present invention is to provide a pharmaceutical composition comprising Ivacaftor having excellent high bioavailability, high solubility which is obtained by using effective and reproducible manufacturing method.

The term "Ivacaftor" as used herein refers to Ivacaftor in the form of free base or in the form of pharmaceutically acceptable salts, crystalline polymorph, solvates, hydrates, esters or in amorphous form or mixture thereof.

Ivacaftor is practically insoluble in water and it shows poor bioavailability after oral administration.

In this invention, the pharmaceutical composition comprises a solid dispersion of Ivacaftor wherein the Ivacaftor is dispersed in a matrix containing at least two polymers and wherein the solid dispersion is prepared by hot-melt extrusion.

The pharmaceutical composition contains a solid dispersion comprising Ivacaftor. The active ingredient is dispersed in a matrix containing polymers, wherein the solid dispersion is prepared by hot-melt extrusion. The expression "solid dispersion" as used herein relates to an active ingredient moleculary dissolved in the solid excipient matrix (solid solution).

Hot-melt extrusion is a manufacturing process where polymeric materials are heated above their glass-transition temperature and mixed with active ingredient, in order to disperse the active ingredient molecularly into the polymer.

According to the one embodiment of the present invention, heating temperatures are used at zones during the hot melt extrusion. The temperature is chosen according to the properties of the polymers and Ivacaftor.

**Table 1: The temperatures of the zones during the hot melt extrusion**

| **Zone 1** | **Zone 2** | **Zone 3** | **Zone 4** | **Zone 5** | **Zone 6** | **Zone 7** | **Zone 8** | **Zone 9** | **Zone 10** |
|---|---|---|---|---|---|---|---|---|---|
| 20 ºC | 60 ºC | 80 ºC | 120 ºC | 180 ºC | 200 ºC | 200 ºC | 80ºC | 60 ºC | 20 ºC |

It was surprisingly found that pharmaceutical composition of high bioavailability, high solubility and showing improved dissolution rate could be obtained that the presence of at least two polymers in a solid dispersion of Ivacaftor wherein obtained by hot-melt extrusion.

In one embodiment, the amount of Ivacaftor is between 15.0% and 55.0%, preferably between 25.0% and 40.0% by weight of the total composition
Suitable polymers are selected from a group comprising hydroxypropyl methyl cellulose acetate succinate, graft copolymer of polyvinyl caprolactam-polyvinyl acetate-polyethylene glycol, homopolymers and copolymers of polyalkylene oxides, polyethylene glycol and polypropylene glycol, homopolymers and copolymers of N-vinyl lactams, N-vinylpyrrolidone, polyvinylpyrrolidone (PVP), Polyvinylpyrrolidone/vinylacetate (PVP/VA), N-vinylcaprolactam, homopolymers and copolymers of acrylic acid and its derivatives, homopolymers and copolymers of methacrylic acid and its derivatives, methylmethacrylate, methylcellulose, ethylcellulose, hydroxymethylcellulose, hydroxyethylcellulose (HEC), hydroxypropylcellulose (HPC), hydroxypropyl methylcellulose (HPMC), carboxymethylcellulose, starch derivatives or mixtures thereof.

To overcome the solubility of Ivacaftor, graft copolymer of polyvinyl caprolactam-polyvinyl acetate-polyethylene glycol was selected as one of the polymers in the hot melt extrusion. It is used to enhance the solubility of poorly water-soluble active ingredients by solid dispersion. The glass transition temperature for graft copolymer of polyvinyl caprolactam-polyvinyl acetate-polyethylene glycol (Soluplus^{®}) is 70 ºC: this low glass transition temperature makes (Soluplus^{®}) a good candidate for active ingredients.

In one embodiment, the polymers are graft copolymer of polyvinyl caprolactam-polyvinyl acetate-polyethylene glycol (Soluplus^{®}) and hydroxypropyl methyl cellulose acetate succinate (HPMCAS).

According to this embodiment, the ratio of weight of hydroxypropyl methyl cellulose acetate succinate to the graft copolymer of polyvinyl caprolactam-polyvinyl acetate-polyethylene glycol is in the range of between 0.30 and 0.60, preferably 0.38 and 0.50. This ratio provides an improved solubility and dissolution profile; thus its bioequivalence is high.

In one embodiment, the pharmaceutical composition comprises at least one pharmaceutically excipient selected from fillers, disintegrants, lubricants, glidants, binders or mixtures thereof.

Suitable fillers are selected from the group comprising lactose, lactose monohydrate, spray-dried lactose monohydrate, anhydrous lactose, microcrystalline cellulose, pregelatinized starch, mannitol, corn starch, maltodextrin, dextrin, dextrose, erytritol, fructose, maltose, sucrose, xylitol, dicalcium phosphate, hydroxypropyl betadex, ammonium alginate, calcium carbonate, calcium phosphate, calcium phosphate dehydrate, neutral pellets, calcium sulfate, cellulose, cellulose acetate, dextrates, dextrin, ethylcellulose, glyceryl palmitostearate, magnesium carbonate, magnesium oxide, maltodextrin, medium chain triglycerides, polydextrose, polymethacrylates, sodium alginate, sodium chloride, sorbitol, starch, sugar sphericals, sulfobutylether beta-cyclodextrin, tragacanth, trehalose, polysorbate 80 or mixtures thereof.

According to one embodiment of the present invention, a preferred filler to be used is microcrystalline cellulose or lactose or mixtures thereof.

According to one embodiment of this invention, the total amount of filler is between 30.0% and 90%, between 55.0% and 70.0 by weight of the total composition.

Suitable binders are selected from the group comprising hydroxypropyl cellulose, hydroxypropyl methyl cellulose, polyethylene glycol, microcrystalline cellulose, polyvinylpyrrolidone, sodium carboxymethyl cellulose, polyvinyl alcohol, pregelatinized starch, glucose, natural gums, sucrose, sodium alginate, carboxy methyl cellulose, methyl cellulose, gelatin, carrageenan, guar gum, carbomer, polymethacrylates, methacrylate polymers, gelatin, alginate, alginic acid, xanthan gum, hyaluronic acid, pectin, polysaccharides, carbomer, poloxamer, polyacrylamide, polyoxyethylene-alkyl ether, polydextrose, polyethylene oxide or mixtures thereof.

Suitable disintegrants are selected from the group comprising calcium carboxymethyl cellulose, crospovidone, cross-linked carboxymethyl cellulose sodium (croscarmellose sodium), starch, sodium carboxymethyl cellulose, carboxymethyl cellulose, docusate sodium, guar gum, polyacryline potassium, sodium alginate, sodium starch glycolate, alginic acid, alginates, ion-exchange resins, magnesium aluminium silica, sodium dodesyl sulphate, poloxamer, sodium glycine carbonate or mixtures thereof.

In one preferred embodiment, the disintegrant is cross-linked carboxymethyl cellulose sodium (croscarmellose sodium).

According to one embodiment, the disintegrant has an amount of at most %5 by weight of the total composition.

Suitable lubricants are selected from the group comprising magnesium stearate, sodium stearyl fumarate, sodium lauryl sulphate, magnesium lauryl sulphate, fumaric acid, glyceryl palmitostearate, hydrogenated natural oils, zinc stearate, calcium stearate, silica, talc, stearic acid, paraffin and mixtures thereof.

In one embodiment, the lubricant can be magnesium stearate.

According to one embodiment, the amount of lubricant is between 0.10% and 5.00%, preferably between 0.25% and 2.50%, more preferably between 0.50% and 1.50% by weight of the total composition.

A preferred surfactant to be used according to the present invention is sodium lauryl sulfate, polysorbate 80, poloxamer, most preferably sodium lauryl sulfate.

According to one embodiment of the present invention, the surfactant is sodium lauryl sulfate.

According to one embodiment, the amount of total surfactant is between 0.05% and 1.50%, preferably between 0.10% and 1.00% by weight of the total composition.

Suitable glidants are selected from a group comprising colloidal silicon dioxide, talc, aluminum silicate or mixtures thereof.

In one preferred embodiment, the glidant is colloidal silicon dioxide.

According to this embodiment, the amount of glidant is between 0.10% and 5.00%, preferably between 0.25% and 2.50%, more preferably between 0.50% and 1.50% by weight of the total composition.

In one preferred embodiment, the pharmaceutical composition comprises Ivacaftor as a active agent, graft copolymer of polyvinyl caprolactam-polyvinyl acetate-polyethylene glycol (Soluplus^{®}) and hydroxypropyl methyl cellulose acetate succinate (HPMCAS) as polymers, croscarmellose sodium as disintegrant, sodium lauryl sulfate as surfactant, colloidal silicon dioxide as glidant, magnesium stearate as lubricant.

According to this preferred embodiment, the composition comprises,
a) 15.0-55.0% by weight Ivacaftor
b) 0.50-5.00% by weight HPMCAS
c) 3.00-9.00% by weight are graft copolymer of polyvinyl caprolactam, polyvinyl acetate. polyethylene glycol (Soluplus^{®})
d) 1.00-5.00% by weight croscarmellose sodium
e) 20.0-40.0% by weight microcrsytalline cellulose
f) 20.0-50.0% by weight lactose
g) 0.10-5.00% by weight colloidal silicon dioxide
h) 0.05-1.50% by weight sodium lauryl sulfate
i) 0.10-5.00% by weight magnesium stearate

The pharmaceutical composition of the invention can be processed into a tablet, capsule form, granules, powders, pellet or unit dose packets.

In one embodiment, the pharmaceutical composition is especially in the tablet form.

According to another embodiment of the present invention, a process for preparing a composition comprising a solid dispersion of Ivacaftor is prepared by hot-melt extrusion:

### Preparation of Extrudate:

a) Mixing Ivacaftor, graft copolymer of polyvinyl caprolactam-polyvinyl acetate-polyethylene glycol (Soluplus^{®}), HPMCAS and sodium lauryl sulfate
b) Melting this mixture in a hot melt extruder for molecular dispersion of Ivacaftor in the polymer to form extrude

### Preparation of Tablet

c) Pulverising and sieving the extrudate
d) Mixing the extrudate powder, croscarmellose sodium, sodium lauryl sulfate, lactose
e) Sieving colloidal silicon dioxide, microcrystalline cellulose and adding to the mixture
f) Adding magnesium stearate to the mixture and mixing
g) Compressing the mixture into tablets

**Example 1:**

| **Ingredients** | | **(%) amount (w/w)** |
|---|---|---|
| Extrudate | Ivacaftor 70.0-90.0% | 25-50 |
| | Soluplus 10.0-16.0% | |
| | HPMCAS 3.0-8.0% | |
| | SLS 0.10-0.70% | |
| Microcrystalline Cellulose | | 20.0-40.0 |
| Lactose | | 20.0-50.0 |
| Sodium Lauryl Sulfate | | 0.20-0.60 |
| Croscarmellose Sodium | | 1.00-5.00 |
| Colloidal Silicon Dioxide | | 0.10.-5.00 |
| Magnesium Stearate | | 0.10-5.00 |
| **TOTAL** | | **100** |

| | | |
|---|---|---|
| Soluplus: graft copolymer of polyvinyl caprolactam,-polyvinyl acetate,-polyethylene glycol | | |

## Claims

1. A pharmaceutical composition comprising a solid dispersion of Ivacaftor wherein the Ivacaftor is dispersed in a matrix containing at least two polymers and wherein the solid dispersion is prepared by hot-melt extrusion.

2. The pharmaceutical composition according to claim 1, wherein the amount of Ivacaftor is between 15.0% and 55.0, preferably between 25.0% and 40.0% by weight of the total composition.

3. The pharmaceutical composition according to claim 1, wherein said at least two polymers are selected from a group comprising hydroxypropyl methyl cellulose acetate succinate, graft copolymer of polyvinyl caprolactam-polyvinyl acetate-polyethylene glycol, homopolymers and copolymers of polyalkylene oxides, polyethylene glycol and polypropylene glycol, homopolymers and copolymers of N-vinyl lactams, Nvinylpyrrolidone, polyvinylpyrrolidone, N-vinylcaprolactam, homopolymers and copolymers of acrylic acid and its derivatives, homopolymers and copolymers of methacrylic acid and its derivatives, methylmethacrylate, methylcellulose, ethylcellulose, hydroxymethylcellulose, hydroxyethylcellulose, hydroxypropylcellulose, hydroxypropyl methylcellulose, carboxymethylcellulose, starch derivatives or mixtures thereof.

4. The pharmaceutical composition according to claim 3, wherein the polymers are hydroxypropyl methyl cellulose acetate succinate and graft copolymer of polyvinyl caprolactam-polyvinyl acetate-polyethylene glycol.

5. The pharmaceutical composition according to claim 4, wherein the weight ratio of hydroxypropyl methyl cellulose acetate succinate to the graft copolymer of polyvinyl caprolactam-polyvinyl acetate-polyethylene glycol is in the range of between 0.35 and 0.60, preferably 0.40 and 0.50.

6. The pharmaceutical composition according to claim 1, wherein the composition further comprising at least one pharmaceutically excipient selected from fillers, disintegrants, lubricants, glidants, binders or mixtures thereof.

7. The pharmaceutical composition according to claim 6, wherein filler is selected from a group of comprising lactose, lactose monohydrate, spray-dried lactose monohydrate, anhydrous lactose, microcrystalline cellulose, pregelatinized starch, mannitol, corn starch, maltodextrin, dextrin, dextrose, erytritol, fructose, maltose, sucrose, xylitol, dicalcium phosphate, hydroxypropyl betadex, ammonium alginate, calcium carbonate, calcium phosphate, calcium phosphate dehydrate, neutral pellets, calcium sulfate, cellulose, cellulose acetate, dextrates, dextrin, ethylcellulose, glyceryl palmitostearate, magnesium carbonate, magnesium oxide, maltodextrin, medium chain triglycerides, polydextrose, polymethacrylates, sodium alginate, sodium chloride, sorbitol, starch, sugar sphericals, sulfobutylether beta-cyclodextrin, tragacanth, trehalose, polysorbate 80 or mixtures thereof.

8. The pharmaceutical composition according to claim 6, wherein the fillers are microcrystalline cellulose or lactose or mixture thereof.

9. The pharmaceutical composition according to any of the preceding claims, wherein the composition comprises,
a) 15.0-55.0% by weight Ivacaftor
b) 0.50-5.00 % by weight HPMCAS
c) 3.00-9.00% by weight are graft copolymer of polyvinyl caprolactam,-polyvinyl acetate-polyethylene glycol
d) 1.00-5.00% by weight croscarmellose sodium
e) 20.0-40.0% by weight microcrsytalline cellulose
f) 20.0-50.0% by weight lactose
g) 0.10-5.00% by weight colloidal silicon dioxide
h) 0.05-1.50% by weight sodium lauryl sulfate
i) 0.10-5.00% by weight magnesium stearate

10. A hot melt extrusion process for preparing the pharmaceutical composition according to claim 9, comprising the following steps;
a) Mixing Ivacaftor, graft copolymer of polyvinyl caprolactam, polyvinyl acetate. polyethylene glycol (Soluplus), HPMCAS and sodium lauryl sulfate
b) Melting this mixture in a hot melt extruder for molecular dispersion of Ivacaftor in the polymer to form extrude
c) Pulverising and sieving the extrudate
d) Mixing the extrudate powder, croscarmellose sodium, sodium lauryl sulfate, lactose
e) Sieving colloidal silicon dioxide, microcrystalline cellulose and adding to the mixture
f) Adding magnesium stearate to the mixture and mixing
g) Compressing the mixture into tablets.

11. The pharmaceutical composition according to any preceding claims, for use in the treatment of cystic fibrosis.
